# EUROPEAN PATENT APPLICATION

(11) **EP 4 389 117 A1**
(43) Date of publication of application: **26.06.2024**
(21) Application number: 22214575.7
(22) Date of filing: 19.12.2022
(51) Int. Cl.: A61K 9/14, A61K 9/48

(54) **PROGESTERONE FORMULATION**

(71) Applicant: Besins Healthcare Distribution FZ-LLC, Dubai (AE)
(72) Inventor: AGNUS, Benoît, 98000 Monaco (MC)
(74) Representative: Nederlandsch Octrooibureau

(57) **Abstract**

The invention relates to a pharmaceutical composition in the form of a suspension comprising wet-medium-milled progesterone particles in a liquid carrier comprising one or more vegetable oils, wherein the progesterone particles have a Dv50 of less than 8 µm, preferably less than 6 µm, more preferably less than 5 µm, as measured by laser light diffractometry. The invention also relates to a method for preparing such a pharmaceutical composition comprising at least a step of subjecting a combination of progesterone and a liquid carrier to a wet-medium-milling operation, so as to produce a suspension of progesterone particles having a Dv50 of less than 8 µm, as measured by laser diffractometry.

## Description

### Field of the Invention

The present invention concerns progesterone compositions and formulations. More in particular, the present invention provides new compositions comprising progesterone particles suspended in an oily carrier, which compositions can suitably be filled in capsules to provide formulations suitable for administration via (amongst others) the oral route. In addition, the present invention provides methods of producing such compositions and formulations, as well as uses of the present compositions and formulations in therapeutic and/or prophylactic methods of treatment.

### Background of the Invention

Progesterone (P) is the natural/endogenous steroid hormone that binds to its specific receptors to induce specific progestational effects. In addition to this, progesterone is able to interfere with the binding sites of other steroids. Therefore the natural hormone exhibits an anti-estrogenic activity, and anti-androgenic activity and also exerts anti-mineralocorticoid effects. Based on this activity profile, progesterone has been approved and used as a medicament, amongst others, as an adjunctive therapy in post-menopausal women with an intact uterus, undergoing oestrogen replacement therapy, as a supplement to the luteal phase during cycles of in vitro fertilization (IVF), and as a means to support pregnancy, i.e. to prevent preterm birth and miscarriage. Medicaments currently approved for these indications typically are provided in the form of filled capsules and tablets containing micronized progesterone for oral administration. It is also available in the form of (intra)vaginal or rectal suppositories or pessaries, topical creams and gels, oil solutions for intramuscular injection, and aqueous solutions for subcutaneous injection

The oral bioavailability of progesterone is very low. This is due to the fact that progesterone is poorly absorbed from the gastrointestinal tract and undergoes massive metabolism in the liver, resulting in almost complete inactivation during the first pass through the liver. It has also been demonstrated that the intestines play a significant role in progesterone metabolism, in part due to metabolism by cytochrome P450 enzymes found in the mucosa of the small intestine and/or due to actions of gut microbiota, which are believed to metabolize progesterone as well.

Because of its poor oral bioavailability, relatively high doses of progesterone are typically used when the oral route of administration is selected in order to achieve significant circulating progesterone levels, which means that levels of its metabolites are relatively high as well.

It wasn't until the 1980's that a suitable oral progesterone formulation was provided for the first time. It was found that micronized preparations of progesterone could solve, to some extent, the low oral bioavailability issue and produce adequate plasma and tissue levels of progesterone. Micronization is the process of reducing the average diameter of the particles of a solid material. By micronizing progesterone, its particles are made smaller and its surface area is increased, thereby enhancing absorption from the intestines.

The first oral formulation comprising micronized progesterone was marketed under the brand name Utrogestan^{®}, developed by LABORATOIRES BESINS-ISCOVESCO and described in French Patent Application FR2408345. The formulation was provided in the form of soft capsules containing micronized progesterone in an oily suspension. The oil which serves as a basis for the oily suspension was peanut oil.

Suspension and partial solubilization of progesterone in an oil containing medium- to long-chain fatty acids has since been shown to aid in the improvement of the bioavailability of oral progesterone. It has been theorized that, due to the lipophilic nature of progesterone, the progesterone molecules and the oil, once released in the gastrointestinal environment, form chylous particles that enter lymph circulation and thus bypass the first-pass effect of liver. Compared to plain milled progesterone, peak levels of progesterone following a single 200 mg oral dose were estimated to be 1.4-fold higher as a consequence of micronization, 1.2-fold as a consequence of the suspension in oil, and 3.2-fold higher due to the combination of micronization and the suspension in oil.

Even though the originally developed Utrogestan^{®} constituted a major step forward in progesterone therapy modalities, the use of peanut oil as the carrier oil proved somewhat disadvantageous, as peanut allergy became increasingly prevalent in the 1980's and 1990's. US 2005/0004076 describes improved variants of the original Utrogestan^{®} formulation, wherein the peanut oil is replaced by one of a select number of alternative vegetable oils, which made it possible to rule out the risks of allergic reactions, while at the same time conserving all of the physicochemical and pharmacokinetic characteristics of the original Utrogestan^{®} formulation, which were the cause of its success. It is explained in US 2005/0004076 that the method for manufacturing the original Utrogestan^{®} formulation on a commercial scale involved a number of highly delicate steps out and that it was crucial that the modification of the formulation would not increase and/or create new manufacturing difficulties.

Based on the invention described in US 2005/0004076, eventually, sunflower oil was selected as the substitute for peanut oil in the next generation Utrogestan^{®} formulation. Pharmacokinetic studies conducted in healthy volunteers have shown that after oral administration of two 100 mg capsules (200mg) of the new formulation, plasma progesterone levels increase to reach a Cₘₐₓ of 13.8ng/ml +/- 2.9ng/ml in 2.2 +/- 1.4 hours. The elimination half-life observed is 16.8+/- 2.3 hours. For comparison, normal progesterone levels during the luteal phase of the menstrual cycle are 6.7 to 22.2 ng/mL. Peak levels of progesterone following a dose of oral progesterone occur after about 1 to 3 hours. The formulation adequately reproduces the anti-estrogenic effect of the natural hormone on the endometrium at the dose of 200 mg daily. It also reproduces the anti-mineralocorticoid effect and has no androgenic action. No side effects have been reported as far as lipids profile, coagulation factors and blood pressure are concerned.

Around 2004, an oral micronized progesterone formulation called Yimaxin was approved in China. According to Wang et al. (Clinical Re-evaluation on Bioequivalence and Relative Bioavailability of Micronized Progesterone Hard Capsule (Yimaxin) and Micronized Progesterone Soft Capsule (Utrogestan) under Vaginal and Oral Administration Routes. Pak J Med Sci. Nov-Dec 2021;37(7):1740-1746), this formulation was developed as an alternative to Utrogestan^{®} without 'oily excipients'. Yimaxin is provided in the form of an ultra-micronized solid dispersion of progesterone in glyceryl monostearate. The manufacturing process comprises, as a first step, the production of a solid dispersion by mixing molten glyceryl monostearate and progesterone followed by spray drying, and, as a second step, subjecting the solid dispersion to jet pulverization. According to Wang et al., Yimaxin results in lower exposure after oral administration than Utrogestan^{®}, but higher exposure after vaginal administration. The study results described by Wang et al. further suggest that Utrogestan^{®} has a more favourable safety profile after oral administration.

Despite the fact that several oral progesterone formulations are now available, there still is room for improvement. For one thing, oral bioavailability is still relatively low for all formulations and there certainly is a long-felt desire to attain further increases. Furthermore, reductions in food effects, interindividual variability, etc., compared to the currently available formulations, would also constitute practically and/or clinically meaningful improvements. It would be particularly desirable to attain any such improvement without having to resort to highly complicated manufacturing steps, without creating new formulation stability issues, etc.

It is an object of the present invention to provide progesterone formulations that are suitable for oral administration to attain any or all of such improved pharmacokinetic, pharmacodynamic and/or safety properties, and that can be manufactured, in the requisite quality/stability, using processing steps that are relatively inexpensive and relatively easy to implement.

### Summary of the Invention

The present invention provides, in a first aspect, a liquid composition comprising wet-medium-milled progesterone particles in a liquid vegetable oil containing carrier, which liquid composition can be filled into capsules to produce unit dosage forms suitable for oral administration to attain improved pharmacokinetic, pharmacodynamic and/or safety properties. In accordance with the invention, the wet-medium-milling of the progesterone is typically done in the (final) liquid carrier. The present inventors established that this technology allows for the production of micronized progesterone particles of significantly smaller size than can be produced by conventional dry milling or grinding techniques. The resulting liquid suspension is immediately ready for filling into suitable capsules, such as soft gelatin capsules.

The present inventors further established that particularly good results can be attained with specific vegetable oils and specific vegetable oil mixtures. Without wishing to be bound by any theory, it is hypothesized that the best performing vegetable oil (mixtures) have a relatively low viscosity, but other properties, such as the fatty acid composition, might influence relevant properties of the liquid composition as well. As is illustrated in the appended experimental results, compositions according to the present invention display particularly favourable dissolution rates in suitable *in vitro* dissolution tests (even in comparison with Utrogestan^{®}), which generally is considered a good proxy for improved oral bioavailability. The oils used in accordance with the invention are not known to be associated with prevalent allergenic responses.

It was furthermore established that the incorporation of a small quantity of soy bean phospholipid (soy bean lecithin) favourably affects the properties of the composition, including storage stability.

Hence, the present invention, in a first aspect provides a pharmaceutical composition in the form of a suspension comprising wet-medium-milled, preferably wet-medium-nanomilled progesterone particles in a liquid carrier comprising one or more vegetable oils.

In a second aspect, a method for preparing a pharmaceutical composition in the form of a suspension comprising wet-medium milled progesterone particles in a liquid carrier, is provided, comprising the following steps:
a. providing a liquid carrier comprising one or more vegetable oils;
b. providing progesterone in the form of a dry powder;
c. combining the progesterone and the liquid carrier; and
d. subjecting the combination of progesterone and liquid carrier to a wet-medium-milling operation, preferably a wet-medium-nanomilling operation.

In a third aspect of the invention, a pharmaceutical composition is provided that is obtainable by the methods of the invention.

In further aspects of the invention, unit dosage forms are provided comprising the pharmaceutical compositions of the invention as well as their therapeutic and prophylactic uses.

These and other aspects of the invention as well as the preferred embodiments thereof will become apparent to those skilled in the art, based on the following detailed description and examples.

### Detailed Description

As will be apparent from the foregoing, a first aspect of the invention concerns a pharmaceutical composition in the form of a suspension comprising wet-medium-milled progesterone particles, preferably wet-medium-nanomilled progesterone particles, in a liquid carrier comprising one or more vegetable oils.

In the context of the present invention the term "progesterone" is used to denote the endogenous human progestogen sex hormone, having the following molecular structure: 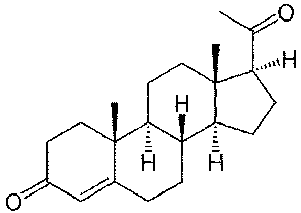 Progesterone is also known as pregnenedione, D4-pregnene-3,20-dione; delta-4-pregnene-3,20-dione; or pregn-4-ene-3,20-dione. As will be apparent to those skilled in the art, based on the present teachings, a fraction of the progesterone contained in the suspension will be present in dissolved form. Whenever, in this document, progesterone is referred to in general, e.g. in relation to quantitative characterizations, this refers to the total progesterone content, i.e. dissolved as well as suspended progesterone. When reference is made to 'progesterone particles' *per se* and/or when particle (size) characteristics are described, this concerns only the suspended progesterone fraction, as will be apparent to those skilled in the art.

The term "pharmaceutical composition", as used herein, refers to a composition comprising one or more active pharmaceutical compounds ('API'), in this case progesterone, optionally in combination with another API, such as an estrogen, and one or more non-pharmacologically active substances, which may be collectively referred to as 'excipients', and wherein all the API's and excipients are of a quality and/or grade that is considered suitable and safe for administration to humans, at the quantities required for the desired therapeutic effect. Typically all API's and excipients are of pharmaceutical grade. The term "pharmaceutical composition" encompasses, but is not limited to, pharmaceutical formulations, which is a term used herein, to denote the pharmaceutical composition in its final form for administration to a human (or animal), such as a unit dose form. In particular, the term "pharmaceutical composition" also encompasses bulk products comprising one or more API's in combination with one or more excipients.

The term "wet-medium-milled" as used herein refers to the product resulting from milling or grinding, which is the process of reducing the particle size of an API, in this case progesterone, in a liquid medium, employing milling media. As will be apparent to those skilled in the art, based on the present teachings, the liquid medium in the present invention typically is an oily liquid, i.e. a liquid comprising one or more oils as the main component.

The term "nanomilled" as used herein thus refers to the product resulting from nanomilling, which is the process of reducing the particle size of an API, in this case progesterone, typically to a (mean, average or median) particle size of around or below 1 µm, in a liquid medium employing milling media. Within the context of the present invention, the term "nanomilling" is deemed synonymous and interchangeable with, in particular, the terms "wet-medium-nanomilling", "liquid-medium-nanomilling" and "(wet) nanogrinding".

In this document, any particle size characteristic of the wet-media-milled progesterone particles is determined using laser light diffractometry and is based on a volume weighted particle size distribution, unless specifically indicated otherwise. Such volume-weighted particle size distributions, in accordance with the invention, are suitably measured using a Malvern Mastersize instrument, such as the Malvern^{®} Mastersizer 2000 with Hydro 2000S module (Malvern 5 Instruments) or an equivalent instrument, typically in accordance with the methods described herein below (see Examples, 'Methodology (General')). As will be understood by those skilled in the art, the term "Dv50" as used herein refers to the point in the size distribution, up to and including which, 50% of the total volume of material in the sample is 'contained'. Similarly, the term "Dv90" refers to the point in the size distribution, up to and including which, 90% of the total volume of material in the sample is contained and the term "Dv10" refers to the point in the size distribution, up to and including which, 10% of the total volume of material in the sample is contained.

In preferred embodiments of the invention, pharmaceutical compositions as defined herein are provided, wherein the progesterone particles have a Dv50 of less than 8 µm, preferably less than 6 µm, less than 5 µm, less than 4 µm, less than 3.5 µm or less than 3 µm, as measured by laser light diffractometry. Furthermore, the Dv50 is typically more than 1 µm, more than 2 µm or more than 2.5 µm.

In preferred embodiments of the invention, pharmaceutical compositions as defined herein are provided, wherein the progesterone particles have a Dv90 of less than 10 µm, preferably less than 9 µm, more preferably less than 8 µm. Furthermore, the Dv90 is typically more than 3 µm, more than 4 µm or more than 5 µm.

In preferred embodiments of the invention, pharmaceutical compositions as defined herein are provided, wherein the progesterone particles have a Dv10 of less than 2 µm, preferably less than 1.5 µm, more preferably less than 1 µm. Furthermore, the Dv10 is typically more than 0.25 µm, more than 0.5 µm or more than 0.75 µm.

As mentioned herein before, the progesterone particles are suspended in a liquid carrier that comprises one or more vegetable oils. As will be understood by those skilled in the art, the term "liquid" refer to the property that a composition is capable of flowing under the influence of force. In accordance with the invention, the carrier typically is liquid at ambient temperature, e.g. at about 20°C.

As used herein, the term "vegetable oil" refers to any fatty substance comprising triglycerides, diglycerides, monoglycerides and combinations thereof, extracted from an oleaginous plant part, such as plant seeds, nuts or fruits containing lipids. As will be understood by those skilled in the art, products that are subjected to conventional (physical) refining and/or purification processes, following their extraction, such as degumming, neutralization, bleaching, winterization, deodorization, etc., are encompassed by the term vegetable oil. Products of chemical reactions involving vegetable oils, such as transesterification products, are usually referred to as "synthetic oils" and are typically not encompassed by the term "vegetable oil". Vegetable oils, typically comprise any one or more of fatty acid triglycerides, diglycerides, monoglycerides and/or combinations thereof, as is known by those skilled in the art. The term "oil" as used herein is readily interchangeable with "lipid" and "fat" and refers to lipophilic high-boiling organic compounds that are liquid at body temperature, i.e. at about 37°C. In preferred embodiments of the inventions, the oils contained in the liquid medium are liquid at body temperatures, i.e. at about 37°C. More preferably, oils are used that are liquid at ambient temperature, i.e. at about 20°C.

In accordance with the present invention, the vegetable oil can comprise saturated, monounsaturated and/or polyunsaturated fatty acids. Saturated fatty acids most abundant in vegetable oils typically include palmitic acid, stearic acid, arachidic acid and lauric acid. Monounsaturated fatty acids most abundant in many vegetable oils include oleic acid and eicosenoic acid. Polyunsatured fatty acids most abundant in vegetable oils include linoleic acid and α-linolenic acid.

In certain embodiments of the present invention, the liquid carrier preferably comprises one or more vegetable oils having a linoleic acid content of at least 10 % w/w, such as at least 25% w/w, at least 40% w/w, at least 50 % w/w, at least 55 % w/w or at least 60 % w/w and/or a linoleic acid content of below 80% w/w, such as below 75% w/w, below 70% w/w or below 65% w/w. In further preferred embodiments of the present invention, the total linoleic acid content of the liquid carrier is at least 10 % w/w, such as at least 25% w/w, at least 40% w/w, at least 50 % w/w, at least 55 % w/w or at least 60 % w/w and/or the total linoleic acid content of the liquid carrier is below 80% w/w, such as below 75% w/w, below 70% w/w or below 65% w/w.

In certain embodiments of the present invention, the liquid carrier preferably comprises one or more vegetable oils having an α linolenic acid content of at least 10 % w/w, such as at least 25% w/w, at least 40% w/w, at least 50 % w/w, at least 55 % w/w or at least 57.5 % w/w and/or an α linolenic acid content of below 80% w/w, such as below 75% w/w, below 70% w/w or below 65% w/w. In further preferred embodiments of the present invention, the total α linolenic acid content of the liquid carrier is at least 10 % w/w, such as at least 25% w/w, at least 40% w/w, at least 50 % w/w, at least 55 % w/w or at least 57.5 % w/w and/or the total α linolenic acid content of the liquid carrier is below 80% w/w, such as below 75% w/w, below 70% w/w or below 65% w/w.

In accordance with the present invention, the liquid carrier preferably comprises one or more vegetable oils having a polyunsaturated fatty acid content of at least of at least 10 % w/w, such as at least 25% w/w, at least 40% w/w, at least 50 % w/w, at least 55 % w/w or at least 60 % w/w and/or a polyunsaturated fatty acid content of below 80% w/w, such as below 75% w/w, below 70% w/w or below 65% w/w. In further preferred embodiments of the present invention, the total polyunsaturated fatty acid content of the liquid carrier is at least 10 % w/w, such as at least 25% w/w, at least 40% w/w, at least 50 % w/w, at least 55 % w/w or at least 60 % w/w and/or the total polyunsaturated fatty acid content of the liquid carrier is below 80% w/w, such as below 75% w/w, below 70% w/w or below 65% w/w.

Gadoleic acid (20:1 n-11), or cis-9-eicosenoic acid, is a fatty acid that is found in relatively low amounts in some vegetable oils. Although the inventors do not wish to be bound by any theory, it is believed that vegetable oils comprising gadoleic acid are particularly preferable for incorporation in the present compositions. Hence, in accordance with certain embodiments of the present invention, the liquid carrier preferably comprises one or more vegetable oils having a gadoleic acid content of at least 0.1% w/w, such as at least 0.5 % w/w, at least 1 % w/w or at least 1.5 % w/w and/or a gadoleic acid content of below 10% w/w, such as below 7.5% w/w, below 5 % w/w, below 4% w/w or below 3% w/w. In further preferred embodiments of the present invention, the total gadoleic acid content of the liquid carrier is at least 0.1% w/w, such as at least 0.5% w/w, at least 1% w/w or at least 1.5% w/w and/or the total gadoleic acid content of the liquid carrier is below 10% w/w, such as below 7.5% w/w, below 5% w/w, below 4% w/w or below 3% w/w.

In another preferred embodiment of the invention, pharmaceutical compositions as defined herein are provided, wherein the one or more vegetable oils comprise:
- gadoleic acid at a level of 0.01 - 30 % w/w;
- linoleic acid at a level of 0.5 - 70 % w/w; or
- α linolenic acid at a level of 0.5 - 70 % w/w.

In preferred embodiments of the invention, pharmaceutical compositions as defined herein are provided, wherein the one or more vegetable oils are selected from the group consisting of rapeseed oil, hemp oil, camelina oil, sesame oil, olive oil, linseed oil, coconut oil, chia oil, sunflower oil and corn germ oil, preferably from the group consisting of sunflower oil, olive oil, linseed oil, chia oil, coconut oil, corn germ oil and camelina oil.

In preferred embodiments of the invention, pharmaceutical compositions as defined herein are provided, wherein the carrier comprises a combination of two or more vegetable oils.

In one such embodiment of the invention, pharmaceutical compositions as defined herein are provided, wherein the carrier comprises a combination of olive oil and linseed oil. In preferred embodiments, pharmaceutical compositions as defined herein are provided, wherein the olive oil and linseed oil are combined in a ratio within the range of 2.5/1-8/1, preferably 3/1-6/1, more preferably 3.5/1-5/1, such as in a ratio of about 4/1. In other preferred embodiments, pharmaceutical compositions as defined herein are provided, wherein the olive oil and linseed oil are combined in a ratio within the range of 1/1-3/1, preferably 1.25/1-2/1, more preferably 1.4/1-1.75/1, most preferably in a ratio of about 1.5/1.

In another preferred embodiment of the invention, pharmaceutical compositions as defined herein are provided, wherein the carrier comprises a combination of corn germ oil and linseed. In preferred embodiments of the invention, pharmaceutical compositions as defined herein are provided, wherein the corn germ oil and linseed oil are present in a ratio within the range of 2.5/1-8/1, preferably 3/1-6/1, more preferably 3.5/1-5/1, most preferably in a ratio of about 4/1.

In preferred embodiments of the invention, pharmaceutical compositions as defined herein are provided, wherein the liquid carrier comprises at least 80 wt.%, based on the total weight of the carrier, of the combination of one or more oils as defined herein, preferably at least 90 wt.%, more preferably at least 95 wt.%, at least 96 wt.%, at least 97 wt.%, at least 98 wt.%, at least 99 wt.% or at least 99.5 wt.%. In other preferred embodiments of the invention, pharmaceutical compositions as defined herein are provided, wherein the liquid carrier (essentially) consists of the combination of one or more vegetable oils as defined herein.

In preferred embodiments of the invention, pharmaceutical compositions as defined herein are provided, wherein the carrier further comprises soy bean lecithin. In a preferred embodiment, soy bean lecithin is present in an amount of more than 0.01% w/w, based on the total weight of the suspension, more than 0.1% w/w, or more than 0.3% w/w and/or in an amount below 15% w/w, based on the total weight of the suspension, below 10% w/w, or below 5% w/w.

In preferred embodiments of the invention, pharmaceutical compositions as defined herein are provided, wherein the carrier does not comprise soy bean lecithin.

In preferred embodiments of the invention, pharmaceutical compositions as defined herein are provided, comprising progesterone in an amount of more than 0.05 mg/ml of liquid carrier, more than 0.1 mg/ml of liquid carrier, more than 0.15 mg/ml of liquid carrier, more than 0.2 mg/ml, more than 0.25 mg/ml of liquid carrier, more than 0.3 mg/ml of liquid carrier, more than 0.35 mg/ml of liquid carrier and/or in an amount below 0.5 mg/ml of liquid carrier or below 0.45 mg/ml of liquid carrier, e.g. about 0.2 or about 0.4 mg/ml of liquid carrier. In further preferred embodiments, progesterone is present in an amount of more than 0.05 mg/ml of vegetable oil(s), more than 0.1 mg/ml of vegetable oil(s), more than 0.15 mg/ml of vegetable oil(s), more than 0.2 mg/ml, more than 0.25 mg/ml of vegetable oil(s), more than 0.3 mg/ml of vegetable oil(s), more than 0.35 mg/ml of vegetable oil(s) and/or in an amount below 0.5 mg/ml of vegetable oil(s) or below 0.45 mg/ml of vegetable oil(s), e.g. about 0.2 or about 0.4 mg/ml of vegetable oil(s). In further preferred embodiments, progesterone is present in an amount of more than 10 % w/w, based on the total weight of the suspension, more than 15% w/w, more than 20% w/w, more than 25% w/w, more than 30% w/w, or more than 35% w/w, and/or in an amount below 50% w/w, based on the total weight of the suspension or below 45% w/w, e.g. about 20% w/w or about 40% w/w.

The pharmaceutical compositions of the present invention have been found to display highly desirable rheology profiles, such as a relatively high resting viscosity combined with shear thinning behaviour. A high resting viscosity is believed to aid in the stability of the suspension, whereas shear thinning behaviour is beneficial with a view to, amongst other things, processing and manufacturing. Viscosity can be measured using a suitable viscometer in a setup that is compatible for viscous (oily) materials. For example, viscosity can be measured using a viscometer from, e.g., Brookfield (Brookfield Viscometer), Lamy Rheoloy (Rheomat RM100), Anton Paar (Rheoplus viscometer), etc., with an appropriate setup. Whenever a viscosity value is defined/quantified in this document, it concerns the viscosity as measured using a Lamy Rheology^{®} Rheomat RM100 viscometer, typically using the set-up and methodology described herein below (see Examples, 'Methodology (General')).

In preferred embodiments of the invention, pharmaceutical compositions as defined herein are provided, wherein the liquid carrier has a viscosity (at ambient temperature), at a shear rate of 1.5 s⁻¹, of at least 50 Pa.s, preferably at least 60 Pa.s, at least 70 Pa.s, at least 80 Pa.s, at least 90 Pa.s or at least 100 Pa.s, and, typically, less than 200 Pa.s, preferably less than 175 Pa.s, less than 150 Pa.s or less than 140 Pa.s.

In further preferred embodiments of the invention, pharmaceutical compositions as defined herein are provided, wherein the liquid carrier has a viscosity (at ambient temperature), at a shear rate of 5 s⁻¹, of at least 15 Pa.s, preferably at least 20 Pa.s, at least 25 Pa.s, at least 30 Pa.s, at least 35 Pa.s or at least 40 Pa.s, and, typically, less than 100 Pa.s, preferably less than 80 Pa.s, less than 70 Pa.s or less than 60 Pa.s.

In further preferred embodiments of the invention, pharmaceutical compositions as defined herein are provided, wherein the liquid carrier has a viscosity (at ambient temperature), at a shear rate of 50 s⁻¹, within the range of 2-20 Pa.s, such as within the range of 4-17.5 Pa.s, within the range of 6-15 Pa.s or within the range of 8-14 Pa.s,

In preferred embodiments of the invention, pharmaceutical compositions as defined herein are presented in unit dosage form, wherein each unit comprises progesterone in an amount of 20 mg or more, such as 50 mg or more, 75 mg or more, 100 mg or more and/or in an amount of 600 mg or less, such as 500 mg or less 400 mg or less or 300 mg or less. In preferred embodiments of the invention, pharmaceutical compositions as defined herein are presented in unit dosage form, wherein each unit comprises progesterone in an amount of about 50 mg, about 100 mg, about 150 mg, about 200 mg, about 250 mg, about 300 mg, about 350 mg or about 400 mg.

The term "unit dosage form" as used herein refers to a single, discrete entity for drug administration, comprising a fixed, pre-determined quantity of the API (or APIs), in association with any suitable pharmaceutical carrier(s) and/or excipient(s). Typically, a unit dosage form comprises the quantity of API (or API's) that is required per administration interval in order to produce the desired therapeutic effect, although situations may be envisaged wherein such quantity is divided over two or even more units, which should be administered together and/or at the same moment. In general, a unit dosage form can be a discrete solid entity, such as a capsule (e.g. a hard or a soft capsule, preferably a soft gelatin capsule, with a solid or liquid fill). Solid formulations, such as a tablet, a caplet, a powder, (encapsulated) pellets, etc., are also envisaged. For the production of solid formulations, the liquid suspension of the invention may, e.g., be used to impregnate an absorbent support presented in the form of powder. This absorbent support may be of the maltodextrin and/or derivatives, silica and/or derivatives, cyclodextrin and/or derivatives or cellulose powder and/or derivatives type, or a combination thereof, or any other pharmaceutical raw material which possesses equivalent properties. The powder thus obtained may then be processed into a hard capsule or tablet, using further excipients, such as binding agents, disintegrating agents, diluents, lubricants, etc. The unit dosage form of the invention can also be an individually packaged amount of the liquid suspension (in an ampoule or sachet).

In accordance with the present invention, the composition is preferably provided in a unit dosage form that is suitable for oral, (intra)vaginal and/or rectal administration, more preferably for oral and/or (intra)vaginal administration.

In preferred embodiments of the invention, pharmaceutical compositions as defined herein are provided, in the form of capsules filled with the combination of wet-medium-milled progesterone particles in a liquid carrier. Preferably, in accordance with the invention, said capsules are of the soft gelatin type. Preferably, a unit dosage form according to the present invention comprises round (sphere shaped) capsule having a diameter within the range of 5 - 35 mm, preferably within the range of 10 -30 mm, more preferably within the range of 12 - 23 mm, most preferably within the range of 15 - 25 mm. Suitable capsules include those referred to in the art as 1 Round, 2 Round, 3 Round, 4 Round, 5 Round, 6 Round, 7 Round, 9 Round, or 15 Round. In other embodiment the capsule is oblong shaped, having a length of within the range of 16 - 20 mm, preferably within the range of 14 - 18 mm, more preferably within the range of 8 - 12 mm, most preferably within the range of 15 - 25 mm, and/or a diameter within the range of 4.0-10.0 mm, preferably within the range of 4.5 - 9.5 mm, more preferably within the range of 5.0 - 9.0 mm, most preferably within the range of 5.5 - 8.5 mm. Suitable capsules include those referred to in the art as Oblong 3, Oblong 4, Oblong 5, Oblong 6, Oblong 7, Oblong 8, Oblong 9.5, Oblong 11 and Oblong 14. In still further embodiments of the invention, the capsules may be oval, such as Oval 2, Oval 3, Oval 4, Oval 5, Oval 6, Oval 7, Oval 8.5, Oval 10, Oval 12 and Oval 16.

In preferred embodiments of the invention, pharmaceutical compositions as defined herein are provided, further comprising an estrogenic component. The term "estrogenic component", as used herein refers to and encompasses substances that are capable of triggering an estrogenic response *in vivo,* as well as precursors that are capable of liberating such an estrogenic component *in vivo* when used in accordance with the present invention. In order for estrogenic components to trigger such a response they normally have to bind to an estrogen receptor, which receptors are found in various tissues within the mammalian body. Exemplary, non-limiting estrogens include estradiol, estriol, estrone, estetrol, ethynyl estradiol and/or derivatives thereof including but not limited to conjugated and esterified estrogens. In particularly preferred embodiments of the invention said estrogenic component is estradiol.

In accordance with preferred embodiments, estradiol may be present in an amount of more than 2 µg/ml of liquid carrier, more than 0.002 mg/ml of liquid carrier, or more than 0.004 mg/ml of liquid carrier, and/or in an amount below 0.2 mg/ml of liquid carrier, below 0.1 mg/ml of liquid carrier, or below 0.05 mg/ml of liquid carrier. In further preferred embodiments, estradiol is present in an amount of more than 2 µg/ml of vegetable oil(s), more than 0.002 mg/ml of vegetable oil(s), or more than 0.004 mg/ml of vegetable oil(s), and/or in an amount below 0.2 mg/ml of vegetable oil(s), below 0.1 mg/ml of vegetable oil(s), or below 0.05 mg/ml vegetable oil(s). In further preferred embodiments, estradiol is present in an amount of more than 0.002% w/w, based on the total weight of the suspension, more than 0.02% w/w, or more than 0.04% w/w, and/or in an amount below 20% w/w, based on the total weight of the suspension, below 10% w/w, or below 5% w/w. In preferred embodiments of the invention, pharmaceutical compositions as defined herein are provided, in unit dosage form, wherein each dosage unit comprises estradiol in an amount of 0.01 mg or more, such as 0.1 mg or more, 1 mg or more, 2 mg or more and/or in an amount of 20 mg or less, such as 15 mg or less 10 mg or less or 5 mg or less. In preferred embodiments of the invention, pharmaceutical compositions as defined herein are provided, in unit dosage form, wherein each dosage unit comprises estradiol in an amount of about 0.01 mg, about 0.1 mg, about 0.3 mg, about 0.5 mg, about 1 mg, about 2 mg, about 4 mg or about 8 mg.

In preferred embodiments of the invention, the liquid suspension comprises more 95% w/w, more than 97.5% w/w, more than 99% w/w, more than 99.5% w/w, more than 99.9% w/w, or (essentially) consists of the combination of progesterone and the liquid carrier as defined herein. In preferred embodiments of the invention, the liquid suspension comprises more 95% w/w, more than 97.5% w/w, more than 99% w/w, more than 99.5% w/w, more than 99.9% w/w, or (essentially) consists of the combination of progesterone and the one or more vegetable oils. In preferred embodiments of the invention, the liquid suspension comprises more 95% w/w, more than 97.5% w/w, more than 99% w/w, more than 99.5% w/w, more than 99.9% w/w, or (essentially) consists of the combination of progesterone, the one or more vegetable oils and soy bean lecithin. In preferred embodiments of the invention, the liquid suspension comprises more 95% w/w, more than 97.5% w/w, more than 99% w/w, more than 99.5% w/w, more than 99.9% w/w, or (essentially) consists of the combination of progesterone, the one or more vegetable oils, soy bean lecithin and optionally one or more further components selected from the group consisting of estrogenic compounds, an anti-oxidants and stabilizers.

A second aspect of the present invention, concerns a method for preparing a pharmaceutical composition in the form of a suspension comprising wet-medium-milled progesterone particles, preferably wet-medium-nanomilled progesterone particles, in a liquid carrier comprising one or more vegetable oils, typically a composition as defined herein before, said method comprising the steps of:
a) providing a liquid medium comprising one or more vegetable oils;
b) providing progesterone in the form of a powder;
c) combining the progesterone and the liquid medium; and
d) subjecting the combination of progesterone and liquid medium to a wet-medium-milling operation, so as to produce a suspension of wet-medium-milled progesterone particles in a liquid carrier comprising one or more vegetable oils.

In certain embodiments of the invention, the liquid medium as provided in step a) of the process is the final liquid carrier of the pharmaceutical composition in accordance with what has been defined herein before. In such embodiments, as will be understood by those skilled in the art, based on the present teachings, the wet-medium-milling step directly yields the final pharmaceutical composition that, e.g., can be filled directly into capsules in order to produce the unit dosage forms defined herein elsewhere. Embodiments are, however, also envisaged wherein the liquid medium as provided in step a) does not comprise all the components/ingredients of the final liquid carrier of the pharmaceutical composition defined herein. In such embodiments, the liquid medium as provided in step a) at least comprises one or more vegetable oils, as defined herein before, while certain other components, such as additional quantities of one or more vegetable oils, which may be the same or different as the vegetable oil(s) comprised in the liquid medium, the soy bean lecithin, and/or any further optional component, may be blended in with the liquid medium during or after step c) and/or with the wet-medium-milled composition during or after step d).

The progesterone as provided in step b) typically takes the form of a powder, but the exact size and/or morphology of the powder particles is not particularly critical. As explained herein elsewhere, the present inventors have established that the wet-medium-milling operation in a liquid (oily) medium allows for a reduction in particle size that cannot be attained using conventional (dry) milling and/or grinding of progesterone powder. More in particular, the particle size of the wet-medium-milled progesterone in accordance with the present inventions is typically below that of standard micronized progesterone products. Hence, as will be understood by those skilled in the art, based on the present teachings, embodiments are envisaged wherein the progesterone as provided in step b) is a (conventional) micronized progesterone product, which can be obtained via various processes that belong to the common general knowledge and/or that can be sourced from commercial suppliers. Embodiments are also envisaged wherein the progesterone as provided in step b) takes the form of a dry, free-flowing powder with coarse particles, wherein the particles size is (well) above that of standard micronized progesterone products.

Step c) of the process can be performed in any conceivable manner. Embodiments are envisaged wherein the liquid medium and progesterone are combined and blended, by some conventional form of stirring or (low-shear) mixing, to produce a homogeneous suspension, which can be fed to the milling apparatus. As will be understood by those skilled in the art, based on the present teachings, the order in which the various components of the liquid medium and progesterone are added to the mixture is not particularly critical. In one embodiment, a mixture comprising all vegetable oil(s) is first produced and, preferably, any further excipient that is to be present in the final liquid suspension, whereafter the progesterone is added to the liquid medium and the resulting mixture is blended. Embodiments are also envisaged wherein the progesterone and liquid medium are separately fed to the milling apparatus, in which case step c) takes place in the milling apparatus, i.e. concurrently with (part of) step d). Embodiments are also envisaged wherein a fraction of the one or more vegetable oils that make up the liquid carrier is added after the wet-medium-milling step. The present inventors established, as explained herein elsewhere, that the ratio of progesterone to liquid medium during the milling operation, affects the extent to which the particle size is reduced under a given set of operating conditions. The higher the (relative) amount of progesterone during the milling operation, the smaller the particles get (or, the quicker a certain extent of particle size reduction is attained). Hence in certain preferred embodiments of the invention, step c) comprises combining the progesterone and the liquid medium in quantities yielding a progesterone (relative) amount of more than 30% w/w, more than 35% w/w, or more than 40% w/w, and the process comprises a subsequent step e) of adding an additional quantity of the one or more vegetable oils (making up the carrier), so as to reduce the progesterone relative amount of the final formulation, as desired, e.g. to any of the relative amounts as defined herein before (in relation to the finished product). Excipients other than the vegetable oils that may optionally be present in accordance with the invention may be added to the suspension obtained after milling as well, even though this is less preferable from a process efficiency standpoint.

As defined herein elsewhere, wet-medium-milling (or wet-medium-nanomillling) refers to the process of reducing the particle size of an API in a liquid medium via grinding using milling media. In this process, the milling media is charged into a chamber containing an agitator. Examples of suitable grinders include disc grinder systems and pin grinding systems. In accordance with the invention, a pin grinding system is preferred as it can typically be operated in high throughput in recirculation mode, which was found to result in particularly favorable particle size characteristics, notably a particularly narrow particle size distribution. An example of a preferred pin grinding system is the Netzsch high-speed mill system Zeta^{®} Type LMZ apparatus, equipped with a DeltaVita^{®} stainless steel pin agitator.

In the wet-medium-milling operation, the API containing suspension is led through the milling chamber, where the agitator rapidly rotates and creates turbulence in the media. As the suspension enters and passes the milling chamber, the shear forces and impaction with milling media break down crystalline structures into smaller particles. The API suspension exits the milling chamber through a screen that separates the suspension, including API (nano)particles, from the milling media. The milling process generates a significant amount of heat. A coolant may pumped around the milling chamber to control the temperature. Hence, in accordance with preferred embodiments of the invention, a method as defined herein is provided, wherein step d) comprises leading the combination of step c) through a milling chamber that is equipped with an agitator and loaded with milling media, while the agitator is operated to cause shear forces and impactions between the milling media and the progesterone particles contained in the suspension.

Various types of milling media are known in the art. In accordance with the invention, the milling media are typically polymeric or ceramic beads. Multiple factors contribute to the selection of milling media type. Ceramic media are denser which means it can impart more energy to the system and reduce particle size for APIs with strong crystalline lattice structures. However, ceramic media may grind the mill itself, leading to increased metal contamination in the suspension. Polymeric media, such as polystyrene media are less dense, which allows them to be run at higher speeds and for longer times without as much metal contamination. The diameter of milling media beads in pharmaceutical processes typically ranges from 0.2 µm to 1 mm. The selection of media size is ultimately dependent on the desired final particle size of the suspension. Smaller media allows one to achieve lower particle sizes and reduces potential contamination compared to larger media. Studies have also shown that smaller media can reduce the time needed to reach a given particle size. In a preferred embodiment of the invention, a method as defined herein is provided, wherein the milling media applied in step d) are yttrium-stabilized zirconium oxide grinding beads with a diameter within the range of 0.2 mm to 1.0 mm. Suitable examples of such milling media include VitaBeads^{®} Nano. The speed of the agitator ultimately controls how much energy is being introduced to the suspension being milled. Higher agitator speeds, e.g. in the thousands of RPMs, result in a faster decrease in particle size because the number of collisions between particles increases. However, issues like temperature sensitivity, and the level of milling impurities affect the possibilities to increase agitator speed. The processing time needed for a suspension is dependent on the desired particle size and the suspension composition. In simpler cases where only a small reduction particle size is needed or an API mills quickly, processing time is relatively short. But in challenging cases where reduced milling speeds are required or an API has a high crystalline lattice energy, processing can take much longer, even multiple days. Increasing the amount of media in the milling chamber can reduce milling time because it results in a higher number of collisions within the chamber. However, the media load must be tailored for each API based on the desired particle size reduction and the properties of the API.

As will be understood by those skilled in the art, based on the present teachings, step d) is carried out in such a manner and for as long as necessary to produce a milled composition with progesterone particles that are within the particle size specifications, typically the Dv10, Dv50 and/or Dv90 values, as provided herein before. Taking into account the present teachings, it will be within the routine capabilities of the person skilled in the art to determine whether or not a given set-up yields a product that complies with said specifications and, thus, to set up the wet-medium-milling process in such a manner to accomplish this.

In preferred embodiments of the invention, a method as defined herein is provided, further comprising the step f) of filling the suspension obtained in step d) or e) as described herein above, into capsules, such as capsules as defined herein before, and, optionally, the step of producing a pharmaceutical kit by packaging a plurality of such capsules in any suitable manner, e.g. in a blister strip that may be inserted in a cardboard box, together with instructions for use.

A further aspect of the present invention concerns a pharmaceutical composition, a unit dosage form and/or kit that is obtainable any of the processes as defined herein before. It is the understanding of the present inventors that the compositions obtained by said processes have the product characteristics defined herein before, but the invention is not limited by any (such) theory.

Yet a further aspect of the invention, concerns pharmaceutical unit dosage forms, in the form of a capsule, preferably a soft gelatin capsule, comprising a pharmaceutical composition comprising wet-medium-milled progesterone suspended in a liquid carrier comprising a vegetable oil, preferably a composition as defined herein before, wherein the pharmaceutical unit dosage form has a USP3 *in vitro* dissolution profile characterized in that more than 70% of progesterone is dissolved at 45 minutes and/or in that more than 20% of progesterone is dissolved after 10 minutes. *In vitro* release characteristics as defined herein, unless specifically stated otherwise, refer to values determined using the USP dissolution system 3, with 250 mL of 0.01 N HCl and 2% SDS as the dissolution medium, at a temperature of 37°C, typically using the set-up and methodology (further) described herein below (see Examples, 'Methodology (General')). In preferred embodiments of the invention, pharmaceutical compositions as defined herein are provided, presented in the form of a filled soft gelatin capsule, characterized in that the amount dissolved in said USP3 *in vitro* dissolution test after 10 minutes is at least 20%, at least 30%, at least 40%, at least 50% or at least 60%. In further preferred embodiments of the invention, pharmaceutical compositions as defined herein are provided, presented in the form of a filled soft gelatin capsule, characterized in that the amount dissolved in said USP3 *in vitro* dissolution test after 20 minutes is at least 40%, at least 45%, at least 50%, at least 55% or at least 60%. In further preferred embodiments of the invention, pharmaceutical compositions as defined herein are provided, presented in the form of a filled soft gelatin capsule, characterized in that the amount dissolved in said USP3 *in vitro* dissolution test after 30 minutes is at least 50%, at least 55%, at least 60%, at least 65% or at least 70%. In further preferred embodiments of the invention, pharmaceutical compositions as defined herein are provided, presented in the form of a filled soft gelatin capsule, characterized in that the amount dissolved in said USP3 *in vitro* dissolution test after 40 minutes is at least 65%, at least 70%, at least 75%, at least 80% or at least 85%. In further preferred embodiments of the invention, pharmaceutical compositions as defined herein are provided, presented in the form of a filled soft gelatin capsule, characterized in that the amount dissolved in said USP3 *in vitro* dissolution test after 60 minutes is at least 80%, at least 85%, at least 90%, at least 92.5 % or at least 95%.

Yet a further aspect of the invention, generally stated, concerns the use of the pharmaceutical compositions of the present invention in the prophylactic and/or therapeutic treatment of a subject in need thereof. More in particular, the invention provides:
- a method of therapeutic and/or prophylactic treatment of a subject in need thereof, said methods comprising the administration to said subject of the pharmaceutical compositions of the present invention;
- a pharmaceutical composition of the present invention, for use in a method of therapeutic and/or prophylactic treatment of subjects in need thereof; and
- the use of a pharmaceutical composition of the present invention, in the manufacture of a medicament for use in the therapeutic and/or prophylactic treatment of subjects in need thereof.

In preferred embodiments of the invention, the therapeutic and/or prophylactic treatment is a method of treating and/or preventing a disease or condition selected from the following list, or the treatment, prevention and/or alleviation of a symptom associated with such a disease or condition:
- luteal insufficiency,
- menstrual irregularity,
- premenstrual syndromes,
- mastodynia,
- benign mastopathies,
- premenopause,
- sterility due to luteal insufficiency,
- disorders due to menopause,
- local contraception,
- repeated abortions in the case of luteal insufficiency,
- premature birth,
- acne,
- alopecia,
- osteoporosis,
- endometrial cancers; and
- epilepsy.

In preferred embodiments, the invention concerns the use of the present pharmaceutical compositions, unit dosage forms and/or capsules in adjunction with estrogen in post-menopausal women with an intact uterus, as hormone replacement therapy (HRT). In other preferred embodiments, the invention concerns the use of the present pharmaceutical compositions, unit dosage forms and/or capsules, for supplementation of the luteal phase during Assisted Reproductive Technology (ART) cycles. In other preferred embodiments, the invention concerns the use of the present pharmaceutical compositions, unit dosage forms and/or capsules for the prevention of preterm birth in women with a singleton pregnancy who have a short cervix (mid-trimester sonographic cervix ≤25 mm) and/or a history of spontaneous preterm birth. In other preferred embodiments, the invention concerns the use of the present pharmaceutical compositions, unit dosage forms and/or capsules for the prevention of endometrial hyperplasia in nonhysterectomized postmenopausal women who are receiving conjugated estrogens tablets. In other preferred embodiments, the invention concerns the use of the present pharmaceutical compositions, unit dosage forms and/or capsules in secondary amenorrhea.

In preferred embodiments of the invention, treatment comprises oral or (intra)vaginal administration of the pharmaceutical composition.

In further preferred embodiments of the invention, the treatment comprises the once daily administration of 1, 2 or 3, preferably 1 or 2 unit dosage forms or capsules as defined herein. In other embodiments of the invention, the treatment comprises the twice daily administration of 1 or 2, preferably 1 unit dosage forms or capsules as defined herein.

Unless otherwise defined, all terms used in disclosing the invention, including technical and scientific terms, have the meaning as commonly understood by one of ordinary skill in the art to which this invention belongs.

"A", "an", and "the" as used herein refer to both singular and plural forms unless the context clearly dictates otherwise. By way of example, "a compartment" refers to one or more than one compartment.

"About" as used herein referring to a measurable value such as a parameter, an amount, a temporal duration, and the like, is meant to encompass variations of +/-10% or less, more preferably +/-5% or less, even more preferably +/-1% or less of and from the specified value, in so far such variations are appropriate to perform in the disclosed invention. However, it is to be understood that the value to which the modifier "about" refers is itself also specifically disclosed.

"Comprise", "comprising", "comprises" and "comprised of" as used herein are synonymous with "include", "including", "includes" or "contain", "containing", "contains" and are inclusive or open-ended terms that specify the presence of what follows, e.g. a component, and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. The skilled person will appreciate that the present invention can incorporate any number of the specific features described above.

Throughout this text, the use of terms in brackets, usually means that the term within brackets specifies a possible option or a possible meaning and should thus not be considered limiting.

Advantages of the invention will become apparent from the following examples, which are given below as mere illustrations, and are non-limitative.

### Brief description of the drawings

Fig 1: schematic view of the assembly used for USP 3 experiments. Dimensions are indicated in millimeters (mm).
Fig. 2: graph showing the dissolution profiles (in percentage dissolved) of progesterone batch 8301189F6 (upper curve) vs Utrogestan^{®} (bottom curve) as a function of time.
Fig 3: graph showing dynamic viscosity values (mPa.s) as a function of shear rate (s⁻¹) of progesterone batch 8301189F6 (upper curve) vs Utrogestan^{®} (bottom curve).

### Examples

### Methodology (general)

### Particle size measurement

Particle size distribution ('PSD') characteristics are determined using a Malvern^{®} Mastersizer 2000 apparatus equipped with a Hydro 2000 SM wet sample dispersion unit, operated with the settings summarized in table 1.

**Table 1 - PSD measurement settings**

| Equipment | MASTERSIZER 2000 MALVERN (APTYS 687) + Hydro 2000SM (APTYS 700) |
|---|---|
| Agitation speed (Hydro 2000SM) | 2000 à 3000 rpm (target: 2000 rpm) |
| Obscuration range | 5% (±0,5%) |
| Refractive index (particle) | 1,540 |
| Absorption index (particle) | 0,01 |
| Dispersing medium | Cyclohexane saturated in micronized progesterone |
| Refractive index (Dispersing medium) | 1,4267 |
| Number of measures | 3 |
| Type of particles | irregular (not spherical) |
| Model | Enhanced |
| Measuring range of the device | 0,020 à 2000,000 µm |
| Results | Volume Distribution |
| Sample preparation | 1. Dilution in the dispersing medium |
| | 2. Magnetic agitation 10 min |
| | 3. Sonication during 20 min |

### Viscosity measurement

Rheology profiles (dynamic viscosity) are determined using a LAMY RHEOLOGY^{®} Rheomat RM100 apparatus, operated with the settings summarized in table 2.

**Table 2 - Viscosity measurement**

| Equipment | Rheomat RM100 LAMY RHEOLOGY^{®} |
|---|---|
| Measuring system | MS-R4 |
| | Mobile : MK-R4 |
| | Bucket: MB-3 |
| | Centering Piece ST-R |
| Temperature | 23 °C (±1.5 °C) |
| Measuring time | 30 seconds |
| Sample weight | 25 g (±1g) |
| Shear rate (s⁻¹) | 1.50; 3.00; 5.00; 10.00; 25.00; 50.00; 62.50; 100.00; 200.00 |

### USP3 dissolution

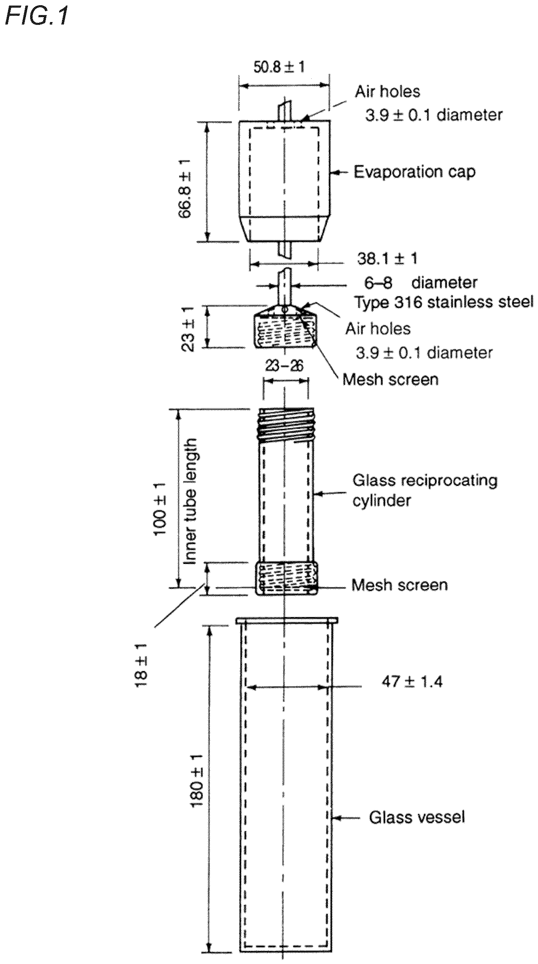

*In vitro* release characteristics are determined using the USP dissolution system 3, schematically depicted in figure 1. The assembly consists of a set of cylindrical, flat-bottomed glass vessels: a set of glass reciprocating cylinders; inert fittings (stainless steel type 316 or other suitable material), and screens that are made of suitable nonsorbing and nonreactive material and that are designed to fit the tops and bottoms of the reciprocating cylinders; and a motor and drive assembly to reciprocate the cylinders vertically inside the vessels; if desired, index the reciprocating cylinders horizontally to a different row of vessels. The vessels are partially immersed in a suitable water bath of any convenient size that permits holding the temperature at 37 ± 0.5°C during the test. No part of the assembly including the environment in which the assembly is placed, contributes significant motion, agitation, or vibration beyond that due to the smooth, vertically reciprocating cylinder. A device us used that allows the reciprocation rate to be selected and maintained at the specified dip rate given in the individual monograph within ± 5%. An apparatus that permits observation of the specimens and reciprocating cylinders is preferable. The vessels are provided with evaporation caps that remain in place for the duration of the test. The components conform to the dimensions shown below, unless otherwise specified in the individual monograph.

Measurements are performed with 250 mL of 0.01 N HCl and 2% SDS as the dissolution medium, at a temperature set at 37°C.

### Example 1: Pharmaceutical Composition in the Form of a wet-medium-milled Suspension According to the Invention

A wet-medium-milled suspension according to the invention is produced (at pilot scale) consisting of a liquid carrier containing olive oil and linseed oil, at a ratio (w/w) of 80/20, and progesterone at a relative amount of 40 wt.%, based on the total weight of the suspension. To this end, refiled USP olive oil (Henry Lamotte Oils GMBH, Bremen, Germany) and virgin linseed oil (Biocoop, France), are blended to form 1.4 kg of homogeneous 80/20 mixture. Micronized Progesterone, with Dv10 = 4.7, Dv50 = 38.4, Dv90 = 74.6, is added to the oil mixture at a quantity resulting in a suspension comprising 40 wt.% of progesterone. For milling a Netzsch Zeta^{®} Labstar LMZ apparatus is used, equipped with a DeltaVita^{®} pin agitator. VitaBeads^{®} nano (0.8 mm) is used as the milling media, at a quantity of 1,656 kg. The milling apparatus is operated at 2,000 rpm. The product temperature is kept below 45°C. The wet-medium-milling operation is continued for 90 minutes. The wet-medium-milled suspension accordingly obtained is referred to as Batch 8301189F6. The main characteristics of this batch are summarized in table 3 below.

**Table 3: Batch 8301189F6 main characteristics**

| Compound name | (%) | (mg) | Function | Reference to standards |
|---|---|---|---|---|
| Active substance | | | | |
| Micronized progesterone | 40.00 | 200.00 | API | Ph. Eur. 3rd ed. |

| Excipients | | | | |
|---|---|---|---|---|
| Olive oil/linsed oil mixture (80/20 ) | 59.60 | 298.00 | Diluent | Ph. Eur. 3rd ed. |
| Soy bean lecithin | 0.40 | 2.00 | Emulsifier | USP 24, NF 19, p.2471 |

### Example 2: Characterization of the wet-medium--milled suspension (vs. Utrogestan^{®})

A part of the product batch produced in example 1 (8301189F6) is used for characterization of physicochemical characteristics, including particle size distribution measurement and viscosity measurement.

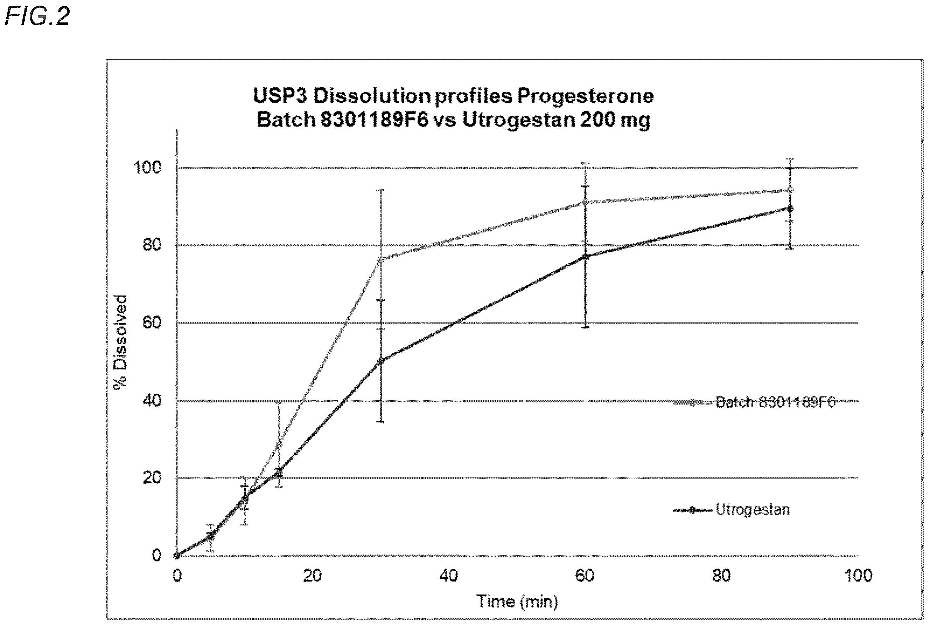

Results of the PSD measurements are summarized in table 4. Results of the dynamic viscosity measurements are summarized in table 5 and plotted in figure 2.

**Table 4: results of PSD measurements**

| | Batch 8301189F6 | Utrogestan^{®} |
|---|---|---|
| Dv10 | 1.005 µm | 8.996 µm |
| Dv50 | 2.663 µm | 18.109 µm |
| Dv90 | 5.545 µm | 29.747 µm |

**Table 5: results of dynamic viscosity measurements (values in mPa.s)**

| | Batch 8301189F6 | | Utrogestan^{®} | |
|---|---|---|---|---|
| Shear rate (s⁻¹) | Up curve | Down curve | Up curve | Down curve |
| 1.50 | 138000 | 107000 | 29000 | 27100 |
| 3.00 | 81300 | 61400 | 17700 | 16200 |
| 5.00 | 56000 | 42200 | 124000 | 11400 |
| 10.00 | 34200 | 26600 | 8437 | 7554 |
| 25.00 | 18800 | 15100 | 5513 | 4901 |
| 50.00 | 12900 | 10300 | 3989 | 3701 |
| 62.50 | 11300 | 9125 | 3621 | 3397 |
| 100.00 | 8374 | 7107 | 3021 | 2891 |
| 200.00 | 5561 | 5323 | 2345 | 2323 |

### Example 3: In Vitro Dissolution Study of the wet-medium-milled suspension vs. Utrogestan^{®}

*In vitro* dissolution studies were carried out with the finished product as produced in example 1 (i.e. Batch 8301189F6 liquid suspension, after filling in gelatin capsules), using a 200 mg Utrogestan^{®} capsule as comparator.

The *in vitro* dissolution profile was determined using the USP3 apparatus, as described above. The seven tanks are placed in a waterbath at constant temperature, and then 1000 ml of dissolution medium are transferred into each of the 7 tanks. One capsule is placed in each of 6 tanks (3 capsules filled with the product of the example 1 batch and 3 of Utrogestan^{®} capsules were tested), and then the baskets are immersed in the dissolution medium at a distance of 25 mm ± 2 mm between the basket and the bottom of the tank. The baskets are stirred, and then a control solution is prepared. At each interval planned (5, 10, 15, 30, 60, 90 minutes), the samples are collected and then analysed by UV spectrophotometry (λ: 248 nm).

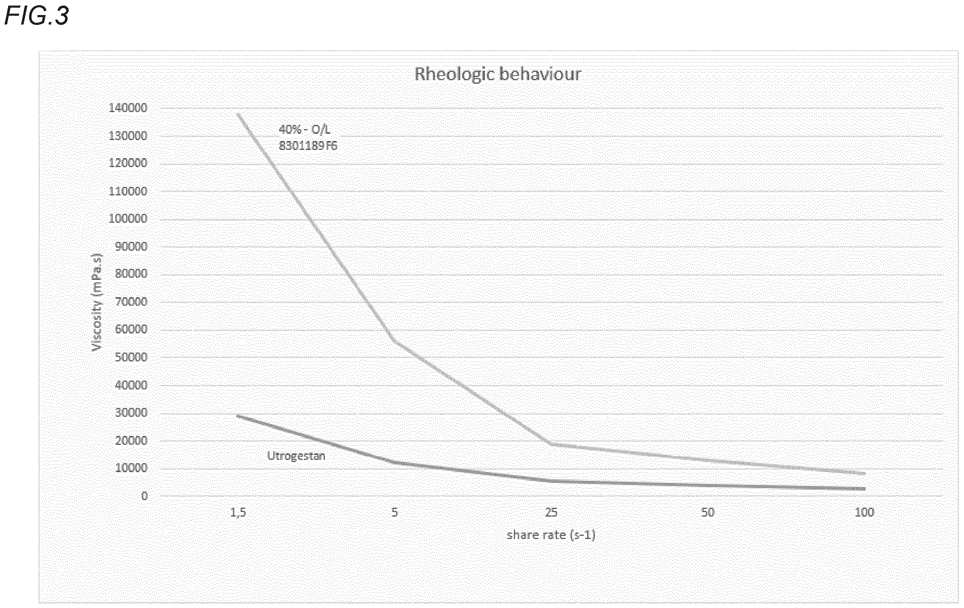

Results of the dissolution experiments are summarized in table 6 and plotted in figure 3.

**Table 6: results of the dissolution experiments**

| | Batch 8301189F6 | | Utrogestan^{®} | |
|---|---|---|---|---|
| Time (min) | Mean (n=3) | Sd | Mean (n=3) | Sd |
| 0 | 0.0 | 0.0 | 0.0 | 0.0 |
| 5 | 4.6 | 3.4 | 5.1 | 0.8 |
| 10 | 14.2 | 6.1 | 14.9 | 2.9 |
| 15 | 28.6 | 10.9 | 21.5 | 1.0 |
| 30 | 76.3 | 17.9 | 50.3 | 15.7 |
| 60 | 91.1 | 10.0 | 77.0 | 18.3 |
| 90 | 94.2 | 8.0 | 89.6 | 10.4 |

The results given in Table 6 and FIG. 3 demonstrate that a capsule filled with the composition according to the invention has a dissolution profile characterized by a higher release rate and/or larger area under the curve, compared to Utrogestan^{®}. These *in vitro* release characteristics imply that the formulations of the invention will also have higher bioavailability of progesterone *in vivo* and that it may be feasible to reduce the (daily) dose of progesterone administered, compared to Utrogestan^{®}, for equal efficacy.

## Claims

1. Pharmaceutical composition in the form of a suspension comprising wet-medium-milled progesterone particles in a liquid carrier comprising one or more vegetable oils, wherein the progesterone particles have a Dv50 of less than 8 µm, preferably less than 6 µm, more preferably less than 5 µm, as measured by laser light diffractometry.

2. Pharmaceutical composition according to claim 1, wherein the progesterone particles have a Dv90 of less than 12 µm, preferably less than 10 µm, more preferably less than 8 µm.

3. Pharmaceutical composition according to claim 1 or 2, wherein the progesterone particles have a Dv10 of less than 2 µm, preferably less than 1.5 µm, more preferably less than 1 µm.

4. Pharmaceutical composition according to any one of the preceding claims, wherein the liquid carrier has a viscosity of at least 80 Pa.s at a shear rate of 1.5 s⁻¹, as measured using a Rheomat RM100 (LAMY RHEOLOGY^{®}) viscometer, at a temperature within the range of 21.5 to 24.5 °C.

5. Pharmaceutical composition according to any one of the preceding claims, wherein the one or more vegetable oils comprise:
- gadoleic acid at a level of 0.01 - 30% w/w;
- linoleic acid at a level of 0.5 - 70% w/w; or
- α linolenic acid at a level of 0.5 - 70% w/w.

6. Pharmaceutical composition according to any one of the preceding claims, wherein the one or more vegetable oils are selected from the group consisting of sunflower oil, olive oil, linseed oil, chia oil, coconut oil, corn germ oil, and camelina oil .

7. Pharmaceutical composition according to any one of the preceding claims, wherein the carrier comprises a combination of two or more vegetable oils.

8. Pharmaceutical composition according to claim 7, wherein the carrier comprises a combination of olive oil and linseed oil.

9. Pharmaceutical composition according to claim 8, wherein the olive oil and linseed oil are combined in a ratio within the range of 8/1 - 2.5/1, preferably 6/1 - 3/1, more preferably 5/1 - 3.5/1, most preferably in a ratio of about 4/1.

10. Pharmaceutical composition according to claim 7, wherein the carrier comprises a combination of corn germ oil and linseed

11. Pharmaceutical composition according to claim 10, wherein the corn germ oil and linseed oil are present in a ratio within the range of 8/1 - 2.5/1, preferably 6/1 - 3/1, more preferably 5/1 - 3.5/1, most preferably in a ratio of about 4/1.

12. Pharmaceutical composition according to any one of the preceding claims, wherein the carrier further comprises soy bean lecithin.

13. Pharmaceutical composition according to any one of the preceding claims, wherein the carrier does not comprise soy bean lecithin.

14. Pharmaceutical composition according to any one of the preceding claims wherein the liquid carrier comprises at least 80 wt.%, based on the total weight of the carrier, of the combination of one or more oils, preferably at least 90 wt.%, more preferably at least 95 wt.%.

15. Pharmaceutical composition according to any one of the preceding claims, comprising 0.1 - 0.8 mg of progesterone per ml of liquid carrier.

16. Pharmaceutical composition according to any one of the preceding claims, in unit dosage form, wherein each dosage unit comprises between 20 mg and 600 mg of progesterone, preferably about 100 mg of progesterone, about 200 mg of progesterone or about 300 mg of progesterone.

17. Pharmaceutical composition according to claim 16, wherein the unit dosage form is a capsule, in particular a soft gelatin capsules, filled with the suspension.

18. Pharmaceutical composition according to any one of the preceding claims, wherein the progesterone is present at a level within the range of 20 - 60% w/w, based on the total weight of the suspension.

19. Pharmaceutical composition according to any one of the preceding claims, for use in the prophylactic and/or therapeutic treatment of a subject in need thereof, wherein said treatment comprises oral or vaginal administration of the composition.

20. Method for preparing a pharmaceutical composition in the form of a suspension comprising wet-medium-milled progesterone particles in a liquid carrier, comprising the following steps:
a) providing a liquid carrier comprising one or more vegetable oils;
b) providing progesterone in the form of a powder;
c) combining the progesterone and the liquid carrier; and
d) subjecting the combination of progesterone and liquid carrier to a wet-medium-milling operation, so as to produce a suspension of progesterone particles having a Dv50 of less than 8 µm, as measured by laser diffractometry.

21. Method according to any one of claims 20, further comprising the step e) of filling the suspension obtained in step d) into a capsule.

22. Pharmaceutical composition obtainable by the method according to claim 20 or 21.

23. Pharmaceutical composition according to any one of claims 1-19 and 22, wherein the composition has a USP3 *in vitro* dissolution profile **characterized in that** the amount dissolved after 20 minutes is at least 40%, as determined in a USP3 *in vitro* dissolution test performed with 250 mL of 0.01 N HCl and 2% SDS as the dissolution medium and at a temperature set at 37°C, after 20 minutes is at least 40%.
